# EUROPEAN PATENT SPECIFICATION

(11) **EP 3 557 258 B1**
(45) Date of publication and mention of the grant of the patent: **03.02.2021**
(21) Application number: 17842289.5
(22) Date of filing: 04.12.2017
(51) Int. Cl.: G01N 33/574

(54) **USE OF IN1-GHRELIN FOR THE DIAGNOSIS OF PROSTATE CANCER AND COLON CANCER**
VERWENDUNG VON IN1-GHRELIN FÜR DIE DIAGNOSE VON PROSTATA KREBS UND DARMKREBS
UTILISATION DE IN1-GHRELIN POUR LA DIAGNOSTIQUE DU CANCER DE LA PROSTATE ET DU CANCER DU COLON

(30) Priority: 16.12.2016 ES 201631606
(43) Date of publication of application: 23.10.2019
(73) Proprietor: Universidad De Córdoba, 14071 Córdoba (ES); Servicio Andaluz de Salud, 41071 Sevilla (ES)
(72) Inventor: LUQUE HUERTAS, Raúl M, 14004 Córdoba (ES); CASTAÑO FUENTES, Justo P, 14004 Córdoba (ES); GAHETE ORTIZ, Manuel D, 14004 Córdoba (ES); IBAÑEZ COSTA, Alejandro, 14004 Córdoba (ES); HORMAECHEA AGULLA, Daniel, 14004 Córdoba (ES); JIMÉNEZ VACAS, Juan Manuel, 14004 Córdoba (ES); MORÁIS SARMENTO BORGES CABRAL, André, 14004 Córdoba (ES); LÓPEZ LÓPEZ, Fernando, 14004 Córdoba (ES); REQUENA TAPIA, Mª José, 14004 Córdoba (ES); GÓMEZ GÓMEZ, Enrique, 14004 Córdoba (ES); CARRASCO VALIENTE, Julia, 14004 Córdoba (ES)
(74) Representative: Hoffmann Eitle
(86) International application number: PCT/ES2017/070797
(87) International publication number: WO 2018/109248

(56) References cited:
- D HORMAECHEA-AGULLA ET AL: "In1-ghrelin splicing variant is over-expressed in prostate cancer where it increases aggressiveness features", EUROPEAN UROLOGY SUPPLEMENTS, vol. 14, no. 2, 1 April 2015 (2015-04-01), page e275, XP55463719,
- ALEJANDRO IBÁÑEZ-COSTA ET AL: "In1-ghrelin splicing variant is overexpressed in pituitary adenomas and increases their aggressive features", SCIENTIFIC REPORTS, vol. 5, no. 1, 4 March 2015 (2015-03-04), XP55463731, DOI: 10.1038/srep08714
- RAUL M. LUQUE ET AL: "In1-ghrelin, a splice variant of ghrelin gene, is associated with the evolution and aggressiveness of human neuroendocrine tumors: Evidence from clinical, cellular and molecular parameters", ONCOTARGET, vol. 6, no. 23, 14 August 2015 (2015-08-14), XP55463716, DOI: 10.18632/oncotarget.4316
- HORMAECHEA-AGULLA DANIEL ET AL: "Ghrelin O-acyltransferase (GOAT) enzyme is overexpressed in prostate cancer, and its levels are associated with patient's metabolic status: Potential value as a non-invasive biomarker", CANCER LETTERS, NEW YORK, NY, US, vol. 383, no. 1, 28 September 2016 (2016-09-28), pages 125-134, XP029785564, ISSN: 0304-3835, DOI: 10.1016/J.CANLET.2016.09.022
- DANIEL HORMAECHEA-AGULLA ET AL: "The oncogenic role of the In1-ghrelin splicing variant in prostate cancer aggressiveness", MOLECULAR CANCER, vol. 16, no. 1, 29 August 2017 (2017-08-29), pages 1-16, XP55463944, DOI: 10.1186/s12943-017-0713-9
- Daniel Hormaechea Agulla: "Functional role and therapeutic potential of the somatostatin and ghrelin systems and their splicing variants in prostate cancer", , 30 May 2017 (2017-05-30), pages 1-134, XP55463938, Spain ISBN: 978-607-15-0504-0 Retrieved from the Internet: URL:http://helvia.uco.es/bitstream/handle/ 10396/14837/2017000001597.pdf?sequence=1&i sAllowed=y [retrieved on 2018-03-29]

## Description

### Field of the Art

The present invention is comprised within the field of biomedicine and provides a diagnostic method for the diagnosis of bladder and/or colon cancer which comprises quantifying the expression levels of In1-ghrelin in plasma, serum, blood and/or urine samples.

### Prior State of the Art

Ghrelin is a hormone synthesized fundamentally by the stomach (Kojima et al., 1999, Nature, 402 (6762): 656-60), although it is also expressed in a wide range of tissues in which it acts as a paracrine or autocrine factor (Lago et al., 2005, Vitam Horm., 71: 405-32.; Tena-Sempere, 2005, Growth Horm IGF Res. 15 (2): 83-8.; Leite-Moreira and Soares, 2007, Drug Discov Today. 12 (7-8): 276-88.; Leontiou et al., 2007, Pituitary, 10 (3): 213- 25.; Leite-Moreira et al., 2008, Vitam Horm., 77: 207-38).

Ghrelin can be acylated by ghrelin O-acyltransferase (GOAT); ghrelin (AG) in this form is the main endogenous ligand of the growth hormone secretagogue receptor (GHS-R) type la (GHS-Rlb). Today, the acylated ghrelin (AG)/GHS-Rlb system is known to perform actions in various tissues, many of them related to metabolic function regulation (Tschop et al., 2000, Nature 407 (6806): 908-13; Horvath et al., 2001, Endocrinology. 142. (10): 4163-9; Nakazato et al., 2001, Nature. 409 (6817): 194-8; van der Lely et al., 2004, Endocr Rev., 25 (3): 426-57).

Human ghrelin is encoded by the *GHRL* gene, the transcription of which gives rise to an immature, 117-residue pre-pro-peptide (pre-pro-ghrelin) which, as mentioned above, can be acylated by the GOAT enzyme, and is subsequently processed in any case by prohormone convertases (PC1, PC7, furin), giving rise to the active hormone (AG) or the theoretically inactive hormone (unacylated ghrelin or UAG) (Zhu et al., 2006, J. Biol Chem., 281 (50): 38867- 70.; Garg, 2007, J Clin Endocrinol Metab., 92 (9): 3396-8).

It is typically considered that human ghrelin gene consisted of 4 coding exons (Ex) and one non-coding Ex (Ex0). The pre-pro-ghrelin signal peptide is encoded in Exl, and the mature ghrelin coding sequence (CDS) is encoded by Ex2 and Ex3 (Kanamoto et al., 2004, Endocrinology, 145 (9): 4144-53.; Nakai et al, 2004, Life Sci., 75 (18): 2193-201). However, recent studies have demonstrated that pre-pro-ghrelin also encodes another peptide, namely, obestatin (Zhang et al., 2005, Science, 310 (5750): 996-9) the tissue expression of which differs from that shown by ghrelin and exhibits specific biological actions (Karaoglu et al., 2009, Mol Cell Biochem., 323 (1-2): 113-8.; Tsolakis et al., 2009, Eur J Endocrinol., 160 (6): 941-9; Volante et al., 2009, J Pathol., Aug; 218(4):458-66). Furthermore, thorough examination of the genomic structure of human GHRL has revealed that this gene actually occupies 7.2 kb of the genomic DNA (Seim et al., 2007. BMC genomics 8:298), and in fact, a new Ex (-1) and several regions of Ex0 and Ex1 have been identified.

The expression of these recently identified exon regions gives rise to multiple tissue-specific transcripts which may lack ghrelin or obestatin and even encode new peptides (Seim I et al., 2009, Clin Exp Pharmacol Physiol., 37(1):125-31.; Seim I et al., 2007, BMC Genomics, 8:298). Pre-pro-ghrelin variants lacking exon 3, and therefore obestatin, have also been described. This isoform is called exon3-deleted-ghrelin and is found in several types of tumors (Jeffery et al., 2002, J. Endocrinol., 172 (3): R7-11; Jeffery et al., 2005b, Endocr Relat Cancer., 12 (4): 839-50.; Yeh et al., 2005, Clin Cancer Res., 11(23): 8295-303). Finally, the occurrence of the intron retention process in the murine ghrelin gene has been described (Kineman et al., 2007, J Mol Endocrinol. 38 (5): 511-21). Specifically, intron-2 retention gives rise to a new mRNA isoform called Inl-ghrelin, which contains Ex2 and 3 but lacks Exl, 4 and 5. Although the translation of In1-ghrelin mRNA has still not been proven, its expression is between 10- and 50-fold greater than the expression of native ghrelin in the pituitary gland and hypothalamus, respectively, and most importantly, the expression level of the mRNA of this isoform is regulated under extreme metabolic conditions (fasting and obesity) (Kineman et al., 2007, J Mol Endocrinol., 38 (5): 511-21).

It has been demonstrated that the levels of mRNA encoding Inl-ghrelin are increased in breast cancer cells and neuroendocrine tumor (NET) and pituitary tumor cells (Gahete et al., 2011. PLoS One. 6 (8): e23302) (Luque et al, 2015. Oncotarget. 14;6(23):19619-33) (Ibáñez-Costa et al., 2015. Sci Rep. 5: 8714). A diagnosis and prognosis of breast cancer, NETs, pituitary tumors, ovarian tumors, endometrial tumors and cervical tumors could be established by means of quantifying the mRNA encoding In1-ghrelin (ES 2 372 337 B1).

Non-invasive diagnostic and/or prognostic methods are preferred over tissue sample analysis because they are easier to obtain and eliminate the risks associated with the surgery required for obtaining tissue samples. Plasma sample analysis is a non-invasive diagnostic method useful for the diagnosis of different types of cancer. For example, the quantification of a microRNA present in plasma was used for the diagnosis of ovarian cancer (Zheng et al., 2013, PLoS One, 8(11): e77853).

Hormaechea-Agulla et al. (Europ. Urol. Suppl., 2015) show that the levels of In1-ghrelin is overexpressed in tumour tissue samples obtained from prostate cancer patients. A drawback of non-invasive diagnostic and/or prognostic methods has been the selection of suitable biomarkers. The authors of the present invention have determined that Inl-ghrelin is a biomarker suitable for the diagnosis and prognosis of cancer.

It has furthermore been demonstrated that the overexpression of Inl-ghrelin is associated with the progression and aggressiveness of neuroendocrine tumors (Luque et al., 2015. Oncotarget. (6(23): 19619-33). The authors of the present invention have developed an siRNA silencing the expression of Inl-ghrelin for the treatment of cancer.

### Description of the Drawings

**Figure 1****:** *Expression levels of Inl-ghrelin in biopsies of patients with prostate cancer (PCa) or controls (NP) determined by means of qPCR.* The data represents the mean ± SEM. The asterisks (*, p<0.05) indicate significant differences between the groups.
**Figure 2****:** *Expression levels of Inl-ghrelin, GOAT and GHS-R1b in biopsies of patients with colon cancer or controls determined by means of qPCR.* The data represents the mean ± SEM.
**Figure 3****:** *Circulating plasma levels of Inl-ghrelin in patients with prostate cancer (PCa) or controls (Control) determined by means of RIA.* The data represents the mean ± SEM. The asterisks (*, p<0.05) indicate significant differences between the groups.
**Figure 4****:** *Effect of the overexpression of Inl-ghrelin and ghrelin on the tumor malignancy of prostate cancer-derived cells.* **A)** Effect of the overexpression of In1-ghrelin and ghrelin on PC-3 and VCaP cell line proliferation. **B)** Effect of the overexpression of In1-ghrelin and ghrelin on PC-3 cell line migration. The data represents the mean ± SEM. The asterisks (*, p<0.05) indicate significant differences with respect to the control (mock).

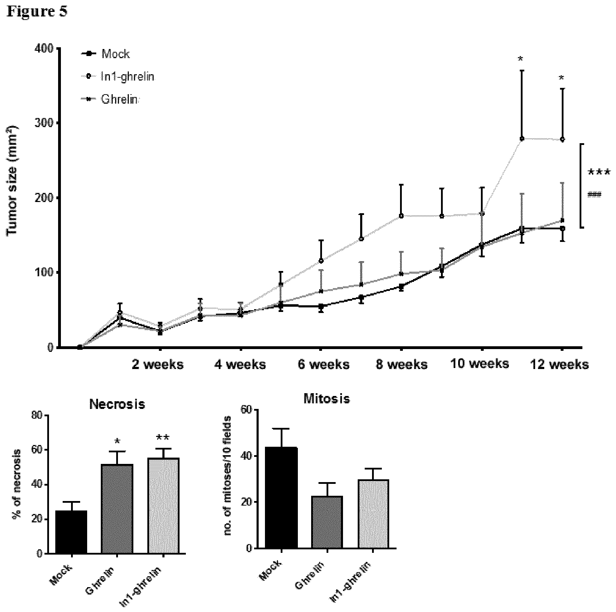

**Figure 5****:** *Effect of the overexpression of Inl-ghrelin and ghrelin on the malignancy of tumors inoculated in athymic mice.* Effect of the overexpression of In1-ghrelin and ghrelin on the growth of tumors induced by the inoculation of PC-3 cells transfected with Inl-ghrelin, ghrelin or controls (mock). Effect of the overexpression of Inl-ghrelin and ghrelin on the necrosis and mitosis of tumors induced by the inoculation of transfected PC-3 cells. The data represents the mean ± SEM. The asterisks or hashes (*, p<0.05; **, p<0.01; *** or ^{###}, p<0.001) indicate significant differences with respect to the control (mock).
**Figure 6****:** *Effect of Inl-ghrelin-specific siRNAs on the tumor malignancy of prostate cancer-derived cellular.* **A)** Validation of the effect of the siRNAs on the expression of Inl-ghrelin in two prostate cancer lines. **B)** Effect of the siRNAs on the proliferation of said cell lines. C) Effect of the siRNA on PSA secretion by LnCaP cells. The data represents the mean ± SEM. The asterisks (*, p<0.05) indicate significant differences with respect to the control (scramble).

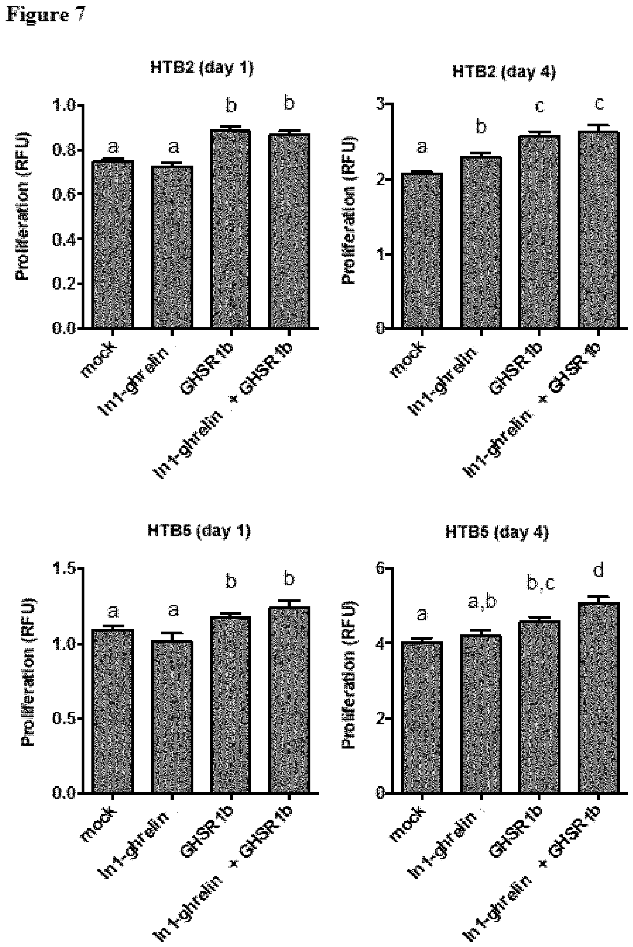

**Figure 7****:** *Effect of the overexpression of Inl-ghrelin and*/*or GHS-Rlb on the proliferation of colon cancer-derived cell lines (HTB2 and HTB5).* The data represents the mean ± SEM. The letters indicate groups between which there are no significant differences.

### Brief Description of the Invention

The present invention relates to a quantification method for quantifying In1-ghrelin as a marker in a diagnostic method and/or a prognostic method for prostate and/or colon cancer. The expression levels of human Inl-ghrelin are consistently increased in plasma, serum, blood and/or urine samples isolated from individuals who have cancer or are at risk of having cancer, compared to reference values.

### Detailed Description of the Invention

### Definitions

Unless specifically mentioned otherwise, the term *"comprises"* is used in the context of this application to indicate that the list of components can include optional aspects that have or have not been mentioned. The term *"comprises"* can also include the concept of *"consists of".*

A *"diagnostic method"* refers to a method in which a patient (or a sample obtained from the patient) is examined or analyzed to obtain differential knowledge about a disease through the signs and symptoms characterizing it. Briefly, the term *"diagnosis"* in the present application refers to the capacity to discriminate between patients who have cancer and patients who do not have cancer. As understood by a person skilled in the art, this discrimination is not intended to be correct in 100% of the analyzed samples. However, it does require a statistically significant amount of the analyzed samples to be correctly classified. The statistically significant amount can be established by a person skilled in the art by using different statistical tools, for example, but without limitation, by means of determining confidence intervals, determining the p-value, Student's t-test or Fisher's discriminant functions. Preferably, confidence intervals are at least 90%, at least 95%, at least 97%, at least 98% or at least 99%. Preferably, the p-value is less than 0.1, 0.05, 0.01, 0.005 or 0.0001. Preferably, the present invention allows correctly detecting the disease in a differential manner in at least 60%, at least 70%, at least 80%, or at least 90% of the subjects of a specific analyzed group or population.

A *"prognostic method"* refers to a method in which the disease or illness, such as cancer, the person suffers is identified based on clinical analyses, and how the future state of health of the individual, i.e., the progression of the disease or illness over time, is predicted based on experiences with other patients suffering from the same disease or illness, such as cancer. The term *"prognosis"* in the present invention also refers to the capacity to detect asymptomatic patients or subjects having a high probability of suffering from a disease or illness, such as cancer, even when they do not have said pathologies or evident manifestations of said pathologies at the time of diagnosis.

The degree of identity existing between two sequences can be determined using conventional methods, for example, by means of standard sequence alignment algorithms which are known in the state of the art, such as BLAST (Altschul S.F. et al. Basic local alignment search tool. J Mol Biol. 1990 Oct 5; 215(3):403-10), for example.

Inl-ghrelin refers to a protein the amino acid sequence of which comprises, or alternatively consists of, SEQ ID NO.: 1 or a fragment thereof. For example, Inl-ghrelin may refer to a fragment of Inl-ghrelin comprising 19 amino acids of the N-terminal end of SEQ ID NO.: 1.

In1-ghrelin is preferably acylated.

Inl-ghrelin mRNA refers to a sequence which comprises, or alternatively consists of, SEQ ID NO.: 2.

GOAT mRNA refers to a sequence which comprises, or alternatively consists of, SEQ ID NO.: 3.

GHS-Rlb mRNA refers to a sequence which comprises, or alternatively consists, SEQ ID NO.: 4.

GOAT refers to a protein the amino acid sequence of which comprises, or alternatively consists of, SEQ ID NO.: 5.

GHS-Rlb refers to a protein the amino acid sequence of which comprises, or alternatively consists of, SEQ ID NO.: 6.

A *"reference sample"* refers to a similar sample obtained from a control subject or individual (patient) who does not have the disease or illness, such as a tumor or cancer, the diagnosis or prognosis of which is to be obtained. A *"reference amount"* or *"reference level"* refers to the absolute amount (or levels) obtained from the reference sample. A *"reference sample"* can also refer to a similar sample obtained from the same subject or individual at an earlier point in time.

A *"reference value"* refers to the set of values used to interpret the results of the diagnostic and/or prognostic tests in a subject or individual (patient). For example, the reference values for a specific test are based on the results of that same test in 95% of the healthy population. The reference values of a test may be different in different groups of people (for example, between women and men). It can also be referred to as *"reference interval", "reference limit",* and *"normal limit".*

The term *"amount"* as it is used herein refers, but is not limited, to the absolute or relative amount of expression products (mRNA and/or protein), as well as to any other value or parameter that relates to them or that can be derived from them. Said values or parameters comprise signal intensity values obtained from any of the physical or chemical properties of the expression products obtained by means of direct measurement. Additionally, said values or parameters include all those values or parameters obtained by means of indirect measurement, for example, by means of any of the measurement systems described in another part of the present document.

The term *"antibody"* as it is used herein refers to immunoglobulin molecules and immunologically active fragments of immunoglobulin molecules, i.e., molecules containing an antigen-binding site that binds specifically (immunoreacts) with, for example, any of the peptide products of Inl-ghrelin, GOAT or GHS-Rlb. Examples of immunologically active fragments include F(ab) and F(ab')₂ fragments which can be generated by treating the antibody with an enzyme such as pepsin. The antibodies can be polyclonal antibodies (they typically include different antibodies targeting different determinants or epitopes) or monoclonal antibodies (targeting a single determinant in the antigen).

The monoclonal antibody can be altered biochemically or by means of genetic manipulation, or it can be synthetic, the antibody possibly lacking, entirely or in parts, fragments or portions that are not required for antigen recognition, such as the peptide products of In1-ghrelin, GOAT or GHS-Rlb, for example, and being substituted by others conferring additional advantageous properties to the antibody.

The antibody can be also recombinant, chimeric, humanized, synthetic or a combination of any of the foregoing. A *"recombinant antibody or polypeptide"* (rAB) is an antibody that has been produced in a host cell which has been transformed or transfected with the nucleic acid which encodes the polypeptide or antigen, or produces the polypeptide or antigen as a result of homologous recombination.

The term *"non-simultaneous"* as it is used herein refers to the acquisition of two or more tissue samples, blood samples (also including plasma and serum samples) or urine samples, where there is a time interval between sample acquisitions which allows performing follow-up of the cancer and/or useful prognosis by means of comparing the amounts of mRNA and/or proteins in the different samples. The time interval is preferably between 1 month and 20 years, such as, for example, between 1 month and 10 years, 1 month and 5 years or 1 month and 1 year. For example, the time interval between sample acquisitions is about 1 month. For example, the time interval between sample acquisitions is about 2 months. For example, the time interval between sample acquisitions is about 3 months. For example, the time interval between sample acquisitions is about 6 months. For example, the time interval between sample acquisitions is about 1 year. For example, the time interval between sample acquisitions is about 1 year and a half. For example, the time interval between sample acquisitions is about 2 years. For example, the time interval between sample acquisitions is about 3 years. For example, the time interval between sample acquisitions is about 5 years.

### Quantification method for quantifying the expression of In1-ghrelin

In a first aspect, the present invention provides a quantification method for quantifying Inl-ghrelin in a sample isolated from the plasma, serum, blood and/or urine of an individual, where said method comprises the step of quantifying the expression levels of In1-ghrelin in a plasma, serum, blood and/or urine sample isolated from an individual.

The quantification method for quantifying the expression of In1-ghrelin according to the first aspect of the present invention is preferably a non-invasive diagnostic method for the diagnosis of cancer which comprises quantifying the expression of In1-ghrelin in the plasma, serum, blood and/or urine of a subject or individual, preferably a subject or individual suspected of having cancer and/or at risk of having cancer.

The diagnostic method of the present invention comprises the steps of:
a. quantifying the expression levels of In1-ghrelin in a plasma, serum, blood and/or urine sample isolated from an individual;
b. comparing the levels obtained in step (a) with a reference value, or with the expression levels of In1-ghrelin obtained in a reference sample; and
c. assigning the patients having expression levels of In1-ghrelin obtained in step (b) that are greater than and statistically significant with respect to the reference value or the levels of the reference sample to the group of individuals at risk of having cancer or group of individuals having cancer, wherein the cancer is prostate and/or colon cancer.

Preferably, the *"reference sample"* of the method according to this embodiment of the first aspect of the present invention (non-invasive diagnostic method) refers to a similar sample obtained from a control subject or individual (patient) who does not have the disease or illness, such as a tumor or cancer, the diagnosis of which is to be obtained.

In a particular embodiment, the quantification method for quantifying the expression of Inl-ghrelin according to the first aspect of the present invention is a prognostic method for the prognosis of prostate and/or colon cancer, said method comprises the steps of:
a. quantifying the expression levels of In1-ghrelin as defined in SEQ ID NO.: 1 in a plasma, serum, blood and/or urine sample isolated from an individual who has prostate and/or colon cancer;
b. comparing the levels obtained in step (a) with a reference value or with the expression levels of In1-ghrelin as defined in SEQ ID NO.: 1 in a reference sample; and
c. assigning the patients having expression levels of In1-ghrelin obtained in step (b) that are greater than and statistically significant with respect to the reference value or the expression levels of the reference sample to the group of individuals with a poor prognosis.

In this embodiment, the *"reference sample"* can be a similar sample obtained from a control subject or individual (patient) who does not have the disease or illness, such as a tumor or cancer, the prognosis of which is to be obtained and/or a similar sample obtained from the same subject or individual at an earlier point in time. When the *"reference sample"* is a similar sample obtained from the same subject or individual at an earlier point in time, the sequence of steps (a)-(b) described above can be performed more than one time, such as two, three, four, five, six, seven, eight, nine, ten, fifteen, twenty or more times, for example, in plasma, serum, blood and/or urine samples obtained from one and the same individual in a non-simultaneous manner. This sequence of steps performed in a non-simultaneous manner provides information that is useful in the prognosis of the disease (cancer) in the patient.

For example, if the expression levels of In1-ghrelin increase over time, it may be indicative of the patient having developed a tumor or of said tumor (which could have already existed beforehand) having increased in malignancy.

The expression levels of In1-ghrelin in a plasma sample are quantified based on the levels of protein present in said plasma sample. Therefore, the levels of the protein as defined in SEQ ID NO.:1 are quantified in steps (a) and (b) of quantifying the expression levels of In1-ghrelin in a plasma sample according to the method of the first aspect of the present invention, in any of the variants thereof.

The amount or concentration of the expression levels can be directly or indirectly measured, preferably in a semi-quantitative or quantitative manner. Direct measurement refers to the measurement of the amount or concentration of the expression products based on a signal obtained directly from the expression products and correlated with the number of molecules of said expression products. Said signal, which can also be referred to as intensity signal, can be obtained by measuring an intensity value of a chemical or physical property of said expression products, for example. Indirect measurement includes the measurement obtained from a secondary component or a biological measurement system (for example, the measurement of cellular responses, ligands, *"labels"* or enzymatic reaction products).

Antibodies can be used to quantify the expression of Inl-ghrelin. These antibodies can be polyclonal or monoclonal antibodies capable of recognizing Inl-ghrelin. The antibodies recognizing Inl-ghrelin can be used to carry out the methods of the present invention by means of an immunoassay. In a preferred embodiment, Inl-ghrelin can be detected, by way of illustration and without limiting the scope of the present invention, by means of incubation with a specific antibody in an immunoassay. The term *"immunoassay"* as it is used herein refers to any analytical technique which is based on the conjugation reaction of an antibody with the obtained sample. Examples of immunoassays known in the state of the art are, for example, but without limitation: immunoblot, enzyme-linked immunosorbent assay (ELISA), radioimmunoassay (RIA), immunohistochemistry or protein microarrays. In another preferred embodiment, Inl-ghrelin and/or the peptide products of Inl-ghrelin can be detected by means of radioimmunoassay.

A *"plasma, serum and*/*or blood sample"* refers to a sample obtained by means of a non-invasive method. A non-limiting example known by a person skilled in the art would be by means of withdrawing a blood sample from an individual using a needle. If a plasma or serum sample is to be obtained, the plasma/serum would be separated from the other blood components by means of centrifugation. The amount of Inl-ghrelin can be analyzed, for example, but without limitation, in fresh plasma, serum and/or blood samples or plasma, serum and/or blood samples that have been frozen and then thawed.

A *"urine sample"* refers to a sample obtained by means of a non-invasive method. A non-limiting example known by a person skilled in the art would be by means of collecting the urine of an individual in a sealable container. The amount of In1-ghrelin can be analyzed, for example, but without limitation, in fresh urine samples or urine samples that have been frozen and then thawed.

The detection of In1-ghrelin in a plasma, serum, blood and/or urine sample in levels that are greater than and/or statistically significant with respect to the reference levels is indicative of said individual having cancer, or of said individual having the risk of having cancer. The terms *"individual", "patient"* or *"subject"* are used interchangeably herein and do not seek to be limiting in any aspect, where the *"individual", "patient"* or *"subject"* can be of any age and sex and have any physical condition.

Therefore, in the method of the present invention, the expression levels of Inl-ghrelin in the plasma, serum, blood and/or urine of a subject can be measured both if the subject has already been diagnosed with the disease (cancer) and if the subject has (still) not been diagnosed with the disease (cancer).

In a preferred embodiment, the diagnostic and/or prognostic method for the diagnosis and/or prognosis of cancer in a patient also comprises combining the quantification of protein as defined in SEQ ID NO.: 1 in the plasma, serum, blood and/or urine with the detection of other biomarker(s), preferably also in plasma, which could improve the precision of said method. For example, but without limitation, the quantification of the expression of Inl-ghrelin could be combined with the detection of PSA (prostate-specific antigen), PCA3 (Prostate CAncer gene 3), GOAT, chromogranin, etc.

### Quantification method for quantifying the expression levels of In1-ghrelin, GOAT and/or GHS-R1b

Also described is a quantification method for quantifying the expression levels of Inl-ghrelin, GOAT and/or GHS-R1b in a bladder and/or colon tissue sample isolated from an individual, where said method comprises the step of quantifying the expression levels of Inl-ghrelin, GOAT and/or GHS-R1b in a bladder and/or colon tissue sample isolated from an individual.

The quantification method for quantifying the expression levels of In1-ghrelin, GOAT and/or GHS-R1b is preferably a diagnostic method for the diagnosis of bladder and/or colon cancer which comprises quantifying the expression levels of In1-ghrelin, GOAT and/or GHS-R1b in a tissue sample from a subject or individual, preferably a subject or individual suspected of having bladder and/or colon cancer and/or at risk of having bladder and/or colon cancer. Therefore, the present disclosure provides a diagnostic method for the diagnosis of bladder and/or colon cancer which comprises the steps of:
a. quantifying the expression levels of In1-ghrelin, GOAT and/or GHS-R1b in a tissue sample isolated from an individual;
b. comparing the levels obtained in step (a) with a reference value or with the expression levels of In1-ghrelin, GOAT and/or GHS-R1b in a reference tissue sample; and
c. assigning the patients having expression levels obtained in step (b) that are greater than and statistically significant with respect to the reference value or the expression levels of Inl-ghrelin, GOAT and/or GHS-R1b of the reference sample to the group of individuals at risk of having bladder and/or colon cancer (or group of individuals having bladder and/or colon cancer).

The *"reference sample"* of the method according to this embodiment of the first aspect of the present disclosure (diagnostic method) preferably refers to a similar sample obtained from a control subject or individual (patient) who does not have the disease or illness, such as a tumor or bladder and/or colon cancer, the diagnosis of which is to be obtained.

In a particular embodiment, the quantification method for quantifying the expression of In1-ghrelin, GOAT and/or GHS-R1b according to the first aspect of the present disclosure is a prognostic method for the prognosis of bladder and/or colon cancer which comprises the steps of:
a. quantifying the expression levels of In1-ghrelin, GOAT and/or GHS-R1b in a tissue sample isolated from an individual who has cancer;
b. comparing the levels obtained in step (a) with a reference value or with the expression levels of In1-ghrelin, GOAT and/or GHS-R1b in a reference tissue sample; and
c. assigning the patients having expression levels obtained in step (b) that are greater than and statistically significant with respect to the reference value or the expression levels of In1-ghrelin, GOAT and/or GHS-R1b of the reference sample to the group of individuals with a poor prognosis.

In this embodiment, the *"reference tissue sample"* can be a similar sample obtained from a control subject or individual (patient) who does not have the disease or illness, such as a tumor or cancer, the prognosis of which is to be obtained and/or a similar sample obtained from the same subject or individual at an earlier point in time. When the *"reference tissue sample"* is a similar sample obtained from the same subject or individual at an earlier point in time, the sequence of steps (a)-(b) described above can be performed more than one time, such as for example two, three, four, five, six, seven, eight, nine, ten, fifteen, twenty or more times, in isolated tissue samples obtained from one and the same individual in a non-simultaneous manner. This sequence of steps performed in a non-simultaneous manner provides information that is useful in the prognosis of the disease (cancer) in the patient.

For example, if the expression levels of In1-ghrelin, GOAT and/or GHS-R1b increase over time, it may be indicative of that subject or patient having developed bladder or colon cancer, or of the cancer which already existed beforehand having progressed unfavorably.

The expression levels of In1-ghrelin, GOAT and/or GHS-R1b in an isolated tissue sample can be quantified based on the levels of protein present in said tissue sample. Therefore, in an embodiment of this second aspect of the present disclosure, the levels of the protein as defined in SEQ ID NO.:1, SEQ ID NO.: 5 and/or SEQ ID NO.: 6 are quantified in steps (a) and (b) of quantifying the expression levels of In1-ghrelin, GOAT and/or GHS-R1b in a tissue sample of the diagnostic and/or prognostic method according to the second aspect of the present disclosure.

The expression levels of In1-ghrelin, GOAT and/or GHS-R1b in a tissue sample can be quantified based on the levels of mRNA present in said tissue sample. Therefore, in an embodiment of this second aspect of the present disclosure, the levels of mRNA as defined in SEQ ID NO.: 2, SEQ ID NO.: 3 and/or SEQ ID NO.: 4 are quantified in steps (a) and (b) of quantifying the expression levels of In1-ghrelin, GOAT and/or GHS-Rlb in a tissue sample of the diagnostic and/or prognostic method according to the second aspect of the present disclosure.

A *"tissue sample"* or *"isolated tissue sample"* includes, but without limitation, cells and/or tissues from a subject, obtained by means of any method known by a person skilled in the art. The tissue sample can be, for example, but without limitation, a biopsy or a fine-needle aspirate. The cells are preferably tumor cells. As described above, the tissue sample is a bladder and/or colon tissue sample.

As described above, the amount or concentration can be measured directly or indirectly, preferably in a semi-quantitative or quantitative manner, as indicated herein above. Direct measurement refers to the measurement of the amount or concentration of the expression products based on a signal obtained directly from the expression products and correlated with the number of molecules of said expression products. Said signal, which can also be referred to as intensity signal, can be obtained by measuring an intensity value of a chemical or physical property of said expression products, for example. Indirect measurement includes the measurement obtained from a secondary component or a biological measurement system (for example, the measurement of cellular responses, ligands, *"labels"* or enzymatic reaction products).

The expression levels can be quantified using any means known in the state of the art. If the expression levels of In1-ghrelin, GOAT and/or GHS-R1b are quantified by means of the quantification of the levels of protein, as described above in the context of the first aspect of the present invention, antibodies can be used to perform said quantification, for example by means of incubation with a specific antibody in an immunoassay, as described above.

If the expression levels of In1-ghrelin, GOAT and/or GHS-R1b are quantified by means of the quantification of the levels of mRNA derived from the transcription of In1-ghrelin, GOAT and/or GHS-R1b genes, said quantification can be performed, by way of illustration and without limiting the scope of the invention, by means of polymerase chain reaction (PCR) amplification, reverse transcription combined with polymerase chain reaction (RT-PCR), reverse transcription combined with ligase chain reaction (RT-LCR), or any other nucleic acid amplification method; DNA chips prepared with oligonucleotides deposited using any mechanism; DNA microarrays prepared with oligonucleotides synthesized *in situ* by means of photolithography or any other mechanism; *in situ* hybridization using specific probes labeled with any labeling method; by means of electrophoresis gels; by means of membrane transfer and hybridization with a specific probe; by means of nuclear magnetic resonance or any other diagnostic imaging technique using paramagnetic nanoparticles or any other type of detectable nanoparticles functionalized with antibodies or by any other means.

Both proteins and mRNA can be quantified, for example, but without limitation, in fresh tissue samples, frozen tissue samples, fixed tissue samples or fixed and paraffin-embedded tissue samples. The use of fixed and paraffin-embedded tissue samples has significant advantages with respect to fresh or frozen tissue samples, since the fixed and paraffin-embedded tissue samples are stable at room temperature, easy to store and there is a large archive of clinical samples available together with their clinical information and disease monitoring.

Also described is the diagnostic and/or prognostic method for the diagnosis and/or prognosis of bladder and/or colon cancer in a patient also comprises combining the quantification of the expression levels of In1-ghrelin, GOAT and/or GHS-R1b in a tissue sample, as described above, with the detection and/or quantification of another/other biomarker(s) which could improve the precision of said method. For example, but without limitation, quantification of the expression levels of In1-ghrelin, GOAT and/or GHS-R1b could be combined with, for example, detection of KRAS (V-Ki-ras2 Kirsten rat sarcoma viral oncogene homolog), and/or with BRAF (v-Raf murine sarcoma viral oncogene homolog B), among others.

In a preferred embodiment, the expression of In1-ghrelin, GOAT and/or GHS-R1b is quantified by determining the amount of mRNA derived from their transcription, where the In1-ghrelin, GOAT and/or GHS-R1b mRNA is quantified by means of RT-PCR using, *in vitro,* at least one of the primers indicated in SEQ ID NO.: 7 (tctgggcttc agtcttctcc), SEQ ID NO.: 8 (gttcatcctc tgccccttct), SEQ ID NO.: 9 (ttgctctttt tccctgctct c), SEQ ID NO.: 10 (actgccacgt ttaggcattc t), SEQ ID NO.: 11 (ggaccagaac cacaagcaaa) and/or SEQ ID NO.: 12 (agagagaagg gagaaggcac a). Preferably, the primers of SEQ ID NO.: 7 and SEQ ID NO.: 8 are used to quantify the amount of mRNA encoding In1-ghrelin; the primers of SEQ ID NO.: 9 and SEQ ID NO.: 10 are used to quantify the amount of mRNA encoding GOAT; and/or the primers of SEQ ID NO.: 11 and SEQ ID NO.: 12 are used to quantify the amount of mRNA encoding GHS-R1b.

Therefore, the present disclosure furthermore provides the *in vitro* use of the primers defined in SEQ ID NO.: 7, SEQ ID NO.: 8, SEQ ID NO.: 9, SEQ ID NO.: 10, SEQ ID NO.: 11 and/or SEQ ID NO.: 12 in the diagnostic and/or prognostic method for the diagnosis and/or prognosis of bladder and/or colon cancer, and/or in the method for obtaining data useful for the diagnosis and/or prognosis of bladder and/or colon cancer according to the second aspect of the present disclosure.

### Method for identifying chemical substances useful in the treatment of cancer

Also described is a method for identifying drugs useful in the treatment of prostate cancer, breast cancer, bladder cancer and/or colon cancer. The method comprises the steps of:
a. establishing prostate, breast, bladder and/or colon cancer cell lines;
b. quantifying the expression levels of In1-ghrelin, GOAT and/or GHS-R1b in said cell lines;
c. administering the "test drug" to the cell lines of (a);
d. quantifying the expression levels of In1-ghrelin, GOAT and/or GHS-R1b in said cell lines after the administration of the "test drug" according to (c); and
e. assigning the "test drug" according to (c) to the group of chemical substances useful in the treatment of prostate cancer, breast cancer, bladder cancer and/or colon cancer when the expression levels of In1-ghrelin, GOAT and/or GHS-R1b quantified in step (d) are lower than and statistically significant with respect to the levels quantified in step (b).

The expression levels of In1-ghrelin, GOAT and/or GHS-R1b in the prostate, breast, bladder and/or colon cancer cell lines according to steps (b) and (d) can be quantified based on the levels of protein and/or the levels of mRNA, as described above.

For example, the expression levels of Inl-ghrelin, GOAT and/or GHS-R1b in the prostate, breast, bladder and/or colon cancer cell lines according to steps (b) and (d) are quantified based on the levels of mRNA, such as based on the levels of mRNA as defined in SEQ ID NO.: 2, SEQ ID NO.: 3 and/or SEQ ID NO.: 4.

Alternatively, the expression levels of In1-ghrelin, GOAT and/or GHS-R1b in the prostate, breast, bladder and/or colon cancer cell lines according to steps (b) and (d) are quantified based on the levels of protein, such as based on the levels of protein as defined in SEQ ID NOs.: 1, 5 and/or 6.

In a preferred embodiment, the first step (a) comprises establishing prostate, breast, bladder and/or colon cancer cell lines. The cancer cell lines can be established by means of any method known by a person skilled in the art. In another preferred embodiment, pre-established cancer cell lines such as, for example, but without limitation PC3, DU145, LNCaP, VCaP, MCF7, MDA-MB-231, CACO-2, HTB-38, HTB-4 and/or HTB-9 cancer cell lines, are used.

The term *"test drug"* refers to a matter with a specific chemical composition made up of its entities: molecules, formula units and atoms. In a preferred embodiment, the test drugs have anticarcinogenic properties. In another preferred embodiment, the test drugs with anticarcinogenic properties are identified in a compound sample library by means of systemic compound identification.

The systemic compound identification can be carried out by means of any method known by a person skilled in the art, such as, for example, but without limitation, *in silico* predictions of antagonists or inhibitory molecules, high-throughput screening assays (high-throughput assays or HTAs), etc.

### siRNA for use in a cancer treatment method

Also described are small interfering RNAs (siRNAs) (hereinafter siRNAs. The siRNAs are useful for the use thereof as a medicinal product, particularly in a cancer treatment method, preferably a prostate cancer, breast cancer, bladder cancer and/or colon cancer treatment method. The siRNAs are preferably useful for the use thereof as a medicinal product, particularly in a cancer treatment method, preferably a prostate cancer, breast cancer, bladder cancer and/or colon cancer treatment method in patients who have been assigned to the group of individuals with cancer or at risk of having cancer by means of the non-invasive diagnostic method described herein.

The *"siRNA"* or *"small interfering RNA"* refers to a type of interfering RNA which is highly specific for the nucleotide sequence of its target mRNA, thereby interfering with the expression of the respective gene. siRNAs intervene in the mechanism referred to as *"RNA interference"* or *"RNAi".*

Specifically, the siRNAs are capable of silencing the mRNA encoding Inl-ghrelin, and thereby repressing its expression. In a preferred embodiment, the target mRNA of the siRNAs is Inl-ghrelin mRNA.

Preferably, one of the siRNAs comprises, or alternatively consists of, SEQ ID NO.: 13 (cacuguuucuggaaggacatt). Therefore, the present invention provides an siRNA comprising, or alternatively consisting of, SEQ ID NO.: 13. Also described is the use of this siRNA as a medicinal product, particularly in a cancer treatment method, preferably a prostate cancer, breast cancer, bladder cancer and/or colon cancer treatment method. Therefore, the present disclosure provides this siRNA for producing a medicinal product, particularly for producing a medicinal product useful in the treatment of cancer, preferably prostate cancer, breast cancer, bladder cancer and/or colon cancer.

Preferably, another one of the siRNAs comprises, or alternatively consists of, SEQ ID NO.: 14 (gagtcctaaacagactgtt). Therefore, the present disclosure provides an siRNA comprising, or alternatively consisting of, SEQ ID NO.: 14. The present disclosure provides the use of this siRNA as a medicinal product, particularly in a cancer treatment method, preferably a prostate cancer, breast cancer, bladder cancer and/or colon cancer treatment method, and preferably in a cancer treatment method in which the cancer is not pituitary cancer. Therefore, the present disclosure provides this siRNA for producing a medicinal product, particularly for producing a medicinal product useful in the treatment of cancer, preferably a cancer which is not pituitary cancer, preferably useful in the treatment of prostate cancer, breast cancer, bladder cancer and/or colon cancer.

The terms *"treatment"* or *"therapy"* as they are used herein refer to a collection of hygienic, pharmacological, surgical and/or physical means the purpose of which is to provide a cure or relief to the diseases and/or symptoms. The terms *"treatment"* or *"therapy"* comprise both prophylactic and curative methods, since they are both aimed at maintaining or restoring health. Regardless of the origin of the illness, disease or disability, the relief thereof by means of the administration of the suitable remedy is understood as therapy or therapeutic use in the context of the present invention.

In another preferred embodiment, the treatment method comprises the use of any of the siRNAs described above combined with one or more chemotherapeutic agents (enzalutamide, abiraterone, etc.), combined with one or more therapeutic antibodies (trastuzumab, etc) and/or combined with the other siRNA of the present disclosure as described above.

### Examples

The prostate cancer samples (n = 52) were obtained from patients having prostate cancer by means of core needle biopsy, whereas the colon cancer samples came from dried samples from patients with said pathologies. Reference tissue samples were obtained from the adjacent tumor-free region or from patients not having of this type of cancer.

The plasma samples were obtained from patients having prostate cancer with different risk levels (n = 30) and the reference samples were obtained from healthy patients (n = 20).

### Example 1: Quantification of the levels of mRNA in prostate and colon cancer samples

The total RNA was isolated from tumor-free and tumor tissue samples using the Allprep DNA/RNA/Protein Mini Kit. The DNA was removed by means of the RNase-Free DNase Kit. The total cell line RNA was extracted with TRIzol. RNA concentration and purity were analyzed using a Nanodrop 2000 spectrophotometer, and the RNA was reverse transcribed using random generic primers and the cDNA First Strand Synthesis kit.

The cDNA was amplified by means of qPCR in parallel with a standard curve which was used to quantify the amount of mRNA in the sample. qPCR was performed using Brilliant III SYBR Green Master Mix and the Stratagene Mx3000p apparatus. The thermal profile consisted of an initial pass at 95°C for 10 minutes, followed by 40 cycles of denaturation (95°C for 30 seconds), annealing (61°C for 1 minute), and extension (72°C for 30 seconds), and finally a melting cycle to verify that only one product was amplified. Inl-ghrelin mRNA was amplified using the primers of SEQ ID NOs.: 7 and 8 (tctgggcttcagtcttctcc and gttcatcctctgccccttct, respectively), GOAT mRNA was amplified using the primers of SEQ ID NOs.: 9 and 10 (ttgctctttttccctgctctc and actgccacgtttaggcattct, respectively) and GHS-R1b mRNA was amplified using the primers of SEQ ID NOs.: 11 and 12 (ggaccagaaccacaagcaaa and agagagaagggagaaggcaca, respectively).

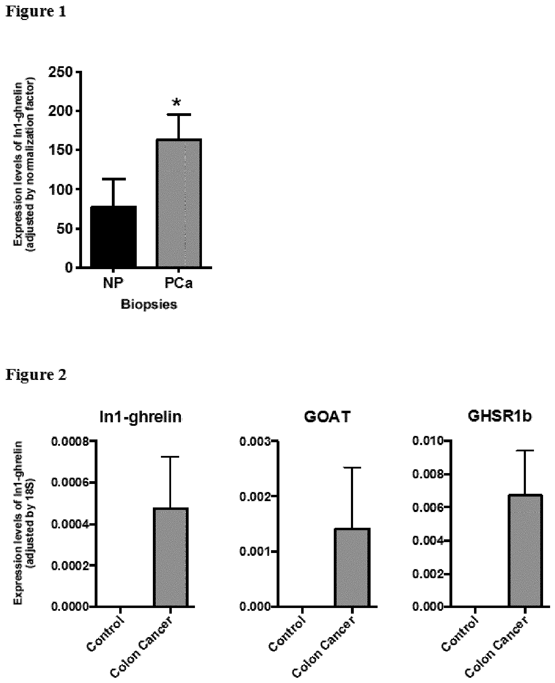

The presence of components associated with the ghrelin system (In1-ghrelin, GOAT and GHS-R1b) was analyzed by means of qPCR. The levels of mRNA in the samples obtained from the patients having prostate cancer (n= 52) were compared with the levels present in the reference samples (n= 12). In1-ghrelin was overexpressed in samples from patients with prostate cancer compared with controls. Figure 1 demonstrates that the levels of In1-ghrelin mRNA are high in samples obtained from patients having prostate cancer when compared with the levels obtained in healthy prostates.

Figure 2 demonstrates that the levels of Inl-ghrelin, GOAT and GHS-Rlb mRNA are high in samples obtained from patients having colon cancer (n=15) when compared with the levels obtained in healthy colon samples (n=1).

### Example 2: Plasma sample protein quantification

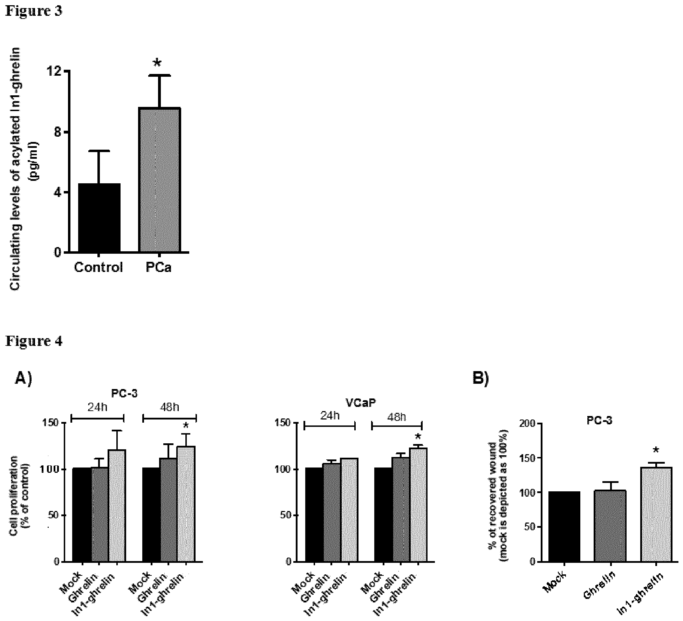

Acylated In1-ghrelin was quantified by means of radioimmunoassay (Catalog Number: RK-032-42; Phoenix Pharmaceuticals, Burlingame, CA, USA) following the manufacturer's instructions. The inventors of the present invention demonstrated that the plasma levels of acylated In1-ghrelin can be detected and that the levels of acylated In1-ghrelin were significantly higher in plasma samples obtained from patients having prostate cancer (n= 30) than in reference plasma samples (n= 20). Figure 3 demonstrates the difference between the levels of acylated Inl-ghrelin in the two types of samples.

### Example 3: Overexpression of in-ghrelin increases prostate cancer cell proliferation and migration

PC-3 and VCaP cell lines were stably transfected with the vector pCDNA3.1 containing an insertion fragment encoding ghrelin or In1-ghrelin, and transformed cells were selected. In more detail, 200,000 cells were seeded in 6-well culture plates and transfected with vectors containing ghrelin, In1-ghrelin or empty vectors, using Lipofectamine 2000 Transfection Reagent, following the manufacturer's instructions. Transfected cells were selected with geneticin.

Cell viability/proliferation was measured by means of Alamar Blue reagent or MTT tetrazolium salt colorimetric assay. For the Alamar Blue assays, cells were seeded at a density of 3,000-5,000 in 96-well plates and deprived of serum for 24 hours. The cells were treated with serum free medium and 10% Alamar Blue for 3 hours. The reduction in Alamar Blue levels was analyzed by means of excitation at 560 nm and detection at 590 nm using the FlexStation III system. For the MTT assays, cells were seeded at a density of 10,000 cells/well in 96-well plates. After culturing for 36 hours, the culture medium was removed and fresh culture medium containing 1% FBS was added. Proliferation was measured after 24, 48 and 72 hours of incubation. Before the measurement, the cells were incubated with a solution containing MTT for 2 hours, the solution was removed and a solution with DMSO, SDS and acetic acid was added and the cells were centrifuged at 150 rpm for 15 minutes. The measurements were taken at 590 nm with FlexStation III.

Cell migration was measured by means of a wound healing assay. 200,000 PC-3 cells were seeded in 6-well culture plates and cultivated to confluence. A scratch (wound) was then made on the surface with a 200 µl pipette tip and the cells were incubated at 37°C for 12 hours. Three separate photographs were taken at 0 and 12 hours after wounding. Wound healing was calculated as the area of the rectangle centered in the photograph taken 12 hours after wounding with respect to the area of the rectangle right after having scratched the surface.

These studies demonstrated that the overexpression of Inl-ghrelin, but not of ghrelin, increased VCaP and PC-3 proliferation after 48 hours (Figure 4A). Furthermore, the overexpression of In1-ghrelin, and not ghrelin, increased PC-3 cell migration (Figure 4B).

### Example 4: Overexpression of in-ghrelin increases xenograft tumor growth

Athymic 10-week old male BALB/cAnNRj-Foxn1nu mice were subcutaneously inoculated on both sides of the torso with 2 x 10⁶ PC-3 cells transfected with vectors containing ghrelin, In1-ghrelin or empty vectors suspended in 100 µl of basement membrane extract. Tumor growth and volume were measured once a week for 3 months with a digital caliper. Each tumor was dissected, fixed and sectioned for histopathological assays. Necrosis and mitosis were examined after staining the samples with hematoxylin and eosin.

It was demonstrated in this study that subcutaneously xenografted tumors were larger if they contained cells that were transfected with a vector containing In1-ghrelin (Figure 5). Furthermore, the histopathological assays demonstrated that the tumors containing cells that were overexpressing In1-ghrelin showed a higher frequency of necrosis without there being changes in the frequency of mitosis (Figure 5).

### Example 5: In1-ghrelin silencing reduced cell proliferation and PSA secretion

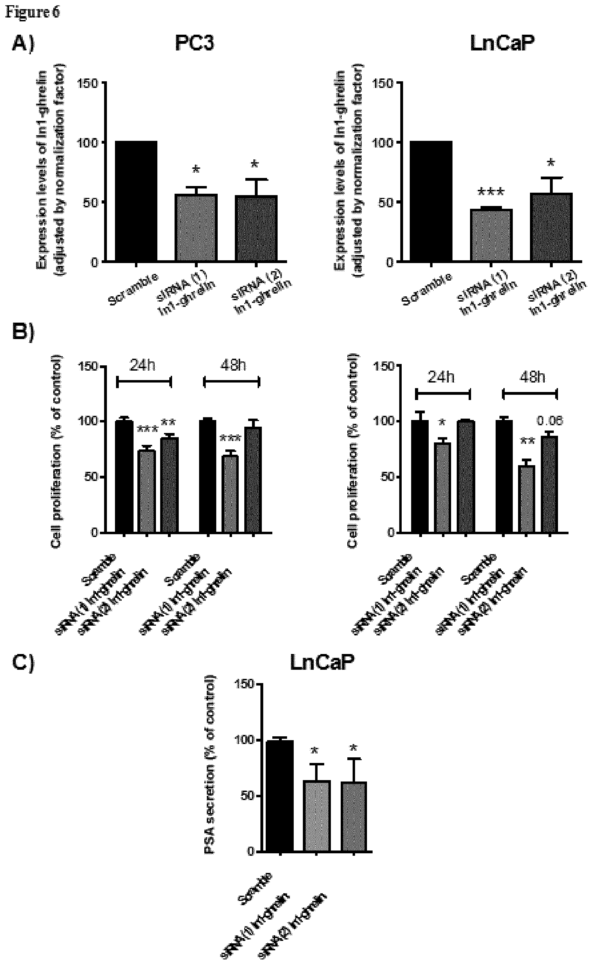

PC-3 and LnCaP cell lines were used for silencing assays. Specifically, 200,000 cells were seeded in 6-well culture plates and the cells were incubated until achieving 70% confluence. The cells were transfected with the siRNA of SEQ ID NO.: 13 (cacuguuucuggaaggacatt), designed to silence the expression of In1-ghrelin [referred to as siRNA (1) in Figure 6], the siRNA of SEQ ID NO.: 14 (gagtcctaaacagactgtt) designed to silence the expression of In1-ghrelin [referred to as siRNA (2) in Figure 6], or with a randomized control using Lipofectamine RNAiMAX. After 48 hours, the cells were collected and seeded to study their proliferation and PSA secretion.

After confirming that the siRNAs had effectively silenced the expression of In1-ghrelin using qPCR (Figure 6A), it was demonstrated that In1-ghrelin silencing reduced PC-3 and LnCaP cell proliferation after 24-48 hours (Figure 6B). Furthermore, In1-ghrelin silencing reduced PSA secretion in LnCaP cells (Figure 6C).

### Example 6: Overexpression of In1-ghrelin and/or GHS-Rlb increases the proliferation of colon cancer-derived cell lines (HTB2 and HTB5).

HTB-2 and HTB-5 cell lines were transiently transfected with the vector pCDNA3.1 containing an insertion fragment encoding In1-ghrelin or GHS-R1b. Specifically, 200,000 cells were seeded in 6-well culture plates and transfected with vectors containing In1-ghrelin and/or GHRS-1b or empty vectors using Lipofectamine 2000 Transfection Reagent following the manufacturer's instructions. Cell viability/proliferation was measured by means of an Alamar Blue reagent colorimetric assay. To that end, cells were seeded at a density of 5,000 in 96-well plates and deprived of serum for 24 hours. The cells were treated with serum-free medium and 10% Alamar Blue for 3 hours. The reduction in Alamar Blue levels was analyzed by means of excitation at 560 nm and detection at 590 nm using the FlexStation III system.

The results of this study demonstrated that the overexpression of In1-ghrelin or GHS-R1b increases the proliferative capacity of colon cancer-derived cells HTB-2 and HTB-5, particularly after 4 days of transfection. Furthermore, this effect seems to be more pronounced when both variants were simultaneously overexpressed, particularly in HTB-5 cells.

## Claims

1. A diagnostic method for the diagnosis of prostate and/or colon cancer in a patient comprising the steps of:
a. quantifying the expression levels of In1-ghrelin as defined in SEQ ID NO.: 1 in a plasma, serum, blood and/or urine sample isolated from an individual suspected of having prostate and/or colon cancer;
b. comparing the levels obtained in step (a) with a reference value or with the expression levels of In1-ghrelin as defined in SEQ ID NO.: 1 in a reference sample; and
c. assigning the individuals having expression levels of In1-ghrelin obtained in step (b) that are greater than and statistically significant with respect to the reference value or the expression levels of the reference sample to the group of individuals at risk of having prostate and/or colon cancer.

2. A prognostic method for the prognosis of prostate and/or colon cancer comprising the steps of:
a. quantifying the expression levels of In1-ghrelin as defined in SEQ ID NO.: 1 in a plasma, serum, blood and/or urine sample isolated from an individual who has prostate and/or colon cancer;
b. comparing the levels obtained in step (a) with a reference value or with the expression levels of In1-ghrelin as defined in SEQ ID NO.: 1 in a reference sample; and
c. assigning the individuals having expression levels of In1-ghrelin obtained in step (b) that are greater than and statistically significant with respect to the reference value or the expression levels of the reference sample to the group of individuals with a poor prognosis.

3. The method according to any of claims 1 to 2, **characterized in that** the quantification of the expression levels of In1-ghrelin as defined in SEQ ID NO.: 1 in a plasma, serum, blood and/or urine sample isolated from an individual is performed by means of an immunoassay, where the immunoassay is an immunoblot, an enzyme-linked immunosorbent assay, radioimmunoassay, immunohistochemistry or protein microarrays.

## Patentansprüche

1. Diagnoseverfahren für die Diagnose von Prostata- und/oder Darmkrebs in einem Patienten umfassend die folgenden Schritte:
a. das Quantifizieren der Expressionsniveaus von In1-Ghrelin, wie in SEQ ID NO.: 1 definiert, in einer Plasma-, Serum-, Blut- und/oder Urinprobe isoliert aus einem Individuum, welches verdächtigt wird, Prostata- und/oder Darmkrebs zu haben;
b. das Vergleichen der in Schritt (a) erhaltenen Niveaus mit einem Referenzwert oder mit den Expressionsniveaus von In1-Ghrelin, wie in SEQ ID NO.: 1 definiert, in einer Referenzprobe; und
c. das Zuordnen der Individuen, welche in Schritt (b) erhaltene Expressionsniveaus von In1-Ghrelin haben, welche größer als und statistisch signifikant in Bezug auf den Referenzwert oder die Expressionsniveaus der Referenzprobe sind, der Gruppe von Individuen, welche gefährdet sind, Prostata- und/oder Darmkrebs zu haben.

2. Prognoseverfahren für die Prognose von Prostata- und/oder Darmkrebs umfassend die folgenden Schritte:
a. das Quantifizieren der Expressionsniveaus von In1-Ghrelin, wie in SEQ ID NO.: 1 definiert, in einer Plasma-, Serum-, Blut- und/oder Urinprobe isoliert aus einem Individuum, welches Prostata- und/oder Darmkrebs hat;
b. das Vergleichen der in Schritt (a) erhaltenen Niveaus mit einem Referenzwert oder mit den Expressionsniveaus von In1-Ghrelin, wie in SEQ ID NO.: 1 definiert, in einer Referenzprobe; und
c. das Zuordnen der Individuen, welche in Schritt (b) erhaltene Expressionsniveaus von In1-Ghrelin haben, welche größer als und statistisch signifikant in Bezug auf den Referenzwert oder die Expressionsniveaus der Referenzprobe sind, der Gruppe von Individuen mit einer schlechten Prognose.

3. Verfahren nach einem der Ansprüche 1 bis 2, **dadurch gekennzeichnet, dass** die Quantifizierung der Expressionsniveaus von In1-Ghrelin, wie in SEQ ID NO.: 1 definiert, in einer Plasma-, Serum-, Blut- und/oder Urinprobe isoliert aus einem Individuum mittels eines Immuntests durchgeführt wird, wobei der Immuntest ein Immunblot, ein enzymgekoppelter Immunosorbenstest, Radioimmuntest, Immunhistochemie oder Proteinmikroarrays ist.

## Revendications

1. Procédé de diagnostic pour le diagnostic du cancer de la prostate et/ou du côlon chez un patient comprenant les étapes de :
a. quantifier les niveaux d'expression d'In1-ghréline tel que définie dans SEQ ID NO : 1 dans un échantillon de plasma, sérum, sang et/ou urine isolé d'un individu soupçonné d'avoir un cancer de la prostate et/ou du côlon ;
b. comparer les niveaux obtenus dans l'étape (a) avec une valeur de référence ou avec les niveaux d'expression d'In1-ghréline tel que définie dans SEQ ID NO : 1 dans un échantillon de référence ; et
c. assigner les individus ayant des niveaux d'expression d'In1-ghréline obtenus dans l'étape (b) qui sont supérieurs à et statistiquement significatifs par rapport à la valeur de référence ou les niveaux d'expression de l'échantillon de référence au groupe d'individus risquant d'avoir un cancer de la prostate et/u du côlon.

2. Procédé de pronostic pour le pronostic du cancer de la prostate et/ou du côlon comprenant les étapes de :
a. quantifier les niveaux d'expression d'In1-ghréline tel que définie dans SEQ ID NO : 1 dans un échantillon de plasma, sérum, sang et/ou urine isolé d'un individu qui a un cancer de la prostate et/ou du côlon ;
b. comparer les niveaux obtenus dans l'étape (a) avec une valeur de référence ou avec les niveaux d'expression d'In1-ghréline tel que définie dans SEQ ID NO : 1 dans un échantillon de référence ; et
c. assigner les individus ayant des niveaux d'expression d'In1-ghréline obtenus dans l'étape (b) qui sont supérieurs à et statistiquement significatifs par rapport à la valeur de référence ou les niveaux d'expression de l'échantillon de référence au groupe d'individus avec un faible pronostic.

3. Procédé selon l'une quelconque des revendications 1 à 2, **caractérisé en ce que** la quantification des niveaux d'expression d'In1-ghréline tel que définie dans SEQ ID NO : 1 dans un échantillon de plasma, sérum, sang et/ou urine isolé d'un individu est exécuté par le biais d'un immunoessai, où l'immunoessai est un immunotransfert, un essai d'immunoabsoption enzymatique, un essai radioimmunologique, immunohistochimie ou des microréseaux de protéines.


### SEQUENCE LISTING

<110> Universidad de Córdoba
<120> Método diagnóstico del cancer
<130> HE-Ref.: 902 685
<160> 14
<170> BiSSAP 1.3.6
<210> 1
   <211> 94
   <212> PRT
   <213> Artificial Sequence
<220>
   <223> In1-ghrelina
<400> 1
   Gly Ser Ser Phe Leu Ser Pro Glu His Gln Arg Val Gln Val Arg Pro
   1               5                   10                  15
   Pro His Lys Ala Pro His Val Val Pro Ala Leu Pro Leu Ser Asn Gln
               20                  25                  30
   Leu Cys Asp Leu Glu Gln Gln Arg His Leu Trp Ala Ser Val Phe Ser
           35                  40                  45
   Gln Ser Thr Lys Asp Ser Gly Ser Asp Leu Thr Val Ser Gly Arg Thr
       50                  55                  60
   Trp Gly Leu Arg Val Leu Asn Arg Leu Phe Pro Pro Ser Ser Arg Glu
   65                  70                  75                  80
   Arg Ser Arg Arg Ser His Gln Pro Ser Cys Ser Pro Glu Leu
                   85                  90
<210> 2
   <211> 354
   <212> RNA
   <213> Artificial Sequence
<220>
   <223> ARNm In1-ghrelina
<400> 2
   atgccctccc cagggaccgt ctgcagcctc ctgctcctcg gcatgctctg gctggacttg      60
   gccatggcag gctccagctt cctgagccct gaacaccaga gagtccaggt gagacctccc     120
   cacaaagccc cacatgttgt tccagccctg ccacttagca accagctctg tgacctggag     180
   cagcagcgcc atctctgggc ttcagtcttc tcccagagca caaaggactc tgggtctgac     240
   ctcactgttt ctggaaggac atgggggctt agagtcctaa acagactgtt tcccccttcc     300
   agcagagaaa ggagtcgaag aagccaccag ccaagctgca gccccgagct ctag           354
<210> 3
   <211> 1663
   <212> RNA
   <213> Artificial Sequence
<220>
   <223> ARNm GOAT
<400> 3
   agacagcctg gttgagatta ctcttggagg aaggcatcag ttaaggacac tggaaaagac      60
   aggctctttt gttctggttc ctaatggagt ggctttggct gttctttctc catcctatat     120
   cgttttacca gggggctgca tttccctttg cacttctctt caattatctc tgcatcatgg     180
   attcattctc cactcgtgcc aggtacctct ttctcctgac tggaggaggt gccctggccg     240
   tggctgccat gggttcctac gccgtgctcg tcttcacccc tgctgtctgc gctgtggctc     300
   tcctctgttc cctggctcct cagcaagtcc acaggtggac cttctgcttt cagatgagct     360
   ggcagacctt gtgtcaccta ggtctgcact acactgagta ttatctgcat gagcctcctt     420
   ctgtgaggtt ctgcatcact ctttcttctc tcatgctctt gacccagagg gtcacgtccc     480
   tctctctgga catttgtgag gggaaagtga aggcagcatc tggaggcttc aggagcagga     540
   gctctttgtc tgagcatgtg tgtaaggcac tgccctattt cagctacttg ctctttttcc     600
   ctgctctcct gggaggctct ctgtgctcct tccagcgatt tcaggctcgt gttcaagggt     660
   ccagtgcttt gcatcccaga cactctttct gggctctgag ctggaggggt ctgcagattc     720
   ttggactaga atgcctaaac gtggcagtga gcagggtggt ggatgcagga gcgggactga     780
   ctgattgcca gcaattcgag tgcatctatg tcgtgtggac cacagctggg cttttcaagc     840
   tcacctacta ctcccactgg atcctggacg actccctcct ccacgcagcg ggctttgggc     900
   ctgagcttgg tcagagccct ggagaggagg gatatgtccc cgatgcagac atctggaccc     960
   tggaaagaac ccacaggata tctgtgttct caagaaagtg gaaccaaagc acagctcgat    1020
   ggctccgacg gcttgtattc cagcacagca gggcttggcc gttgttgcag acatttgcct    1080
   tctctgcctg gtggcatgga ctccatccag gacaggtgtt tggtttcgtt tgctgggccg    1140
   tgatggtgga agctgactac ctgattcact cctttgccaa tgagtttatc agatcctggc    1200
   cgatgaggct gttctataga accctcacct gggcccacac ccagttgatc attgcctaca    1260
   tcatgctggc tgtggaggtc aggagtctct cctctctctg gttgctctgt aattcgtaca    1320
   acagtgtctt tcccatggtg tactgtattc tgcttttgct attggcgaag agaaagcaca    1380
   aatgtaactg acatctttcc ctggctttca ccttaaacac ccaggaaaca ttttagaaag    1440
   tgccagatga cgcatcgata atgcatacat ttgaactttt ccataataaa attttttcta    1500
   aagttttgga tggatatacc cagtgacttg cagtaggaat ttgtgtatct actttagcaa    1560
   gctattctgc tggtactgag gatacaaaaa ggaaaaagat gtggtttctt cccttaaggt    1620
   ttacagtcta gatgtggaag caaagattaa gtaaagtgat aca                      1663
<210> 4
   <211> 914
   <212> RNA
   <213> Artificial Sequence
<220>
   <223> ARNm GHS-R1b
<400> 4
   cagcgcagtc acgtcccaga gcctgttcag ctgagccggc agcatgtgga acgcgacgcc      60
   cagcgaagag ccggggttca acctcacact ggccgacctg gactgggatg cttcccccgg     120
   caacgactcg ctgggcgacg agctgctgca gctcttcccc gcgccgctgc tggcgggcgt     180
   cacagccacc tgcgtggcac tcttcgtggt gggcatcgct ggcaacctgc tcaccatgct     240
   ggtggtgtcg cgcttccgcg agctgcgcac caccaccaac ctctacctgt ccagcatggc     300
   cttctccgat ctgctcatct tcctctgcat gcccctggac ctcgttcgcc tctggcagta     360
   ccggccctgg aacttcggcg acctcctctg caaactcttc caattcgtca gtgagagctg     420
   cacctacgcc acggtgctca ccatcacagc gctgagcgtc gagcgctact tcgccatctg     480
   cttcccactc cgggccaagg tggtggtcac caaggggcgg gtgaagctgg tcatcttcgt     540
   catctgggcc gtggccttct gcagcgccgg gcccatcttc gtgctagtcg gggtggagca      600
   cgagaacggc accgaccctt gggacaccaa cgagtgccgc cccaccgagt ttgcggtgcg     660
   ctctggactg ctcacggtca tggtgtgggt gtccagcatc ttcttcttcc ttcctgtctt     720
   ctgtctcacg gtcctctaca gtctcatcgg caggaagctg tggcggagga ggcgcggcga     780
   tgctgtcgtg ggtgcctcgc tcagggacca gaaccacaag caaaccgtga aaatgctggg     840
   tgggtctcag cgcgcgctca ggctttctct cgcgggtcct atcctctccc tgtgccttct     900
   cccttctctc tgag                                                       914
<210> 5
   <211> 435
   <212> PRT
   <213> Artificial Sequence
<220>
   <223> GOAT
<400> 5
   Met Glu Trp Leu Trp Leu Phe Phe Leu His Pro Ile Ser Phe Tyr Gln
   1               5                   10                  15
   Gly Ala Ala Phe Pro Phe Ala Leu Leu Phe Asn Tyr Leu Cys Ile Met
               20                  25                  30
   Asp Ser Phe Ser Thr Arg Ala Arg Tyr Leu Phe Leu Leu Thr Gly Gly
           35                  40                  45
   Gly Ala Leu Ala Val Ala Ala Met Gly Ser Tyr Ala Val Leu Val Phe
       50                  55                  60
   Thr Pro Ala Val Cys Ala Val Ala Leu Leu Cys Ser Leu Ala Pro Gln
   65                  70                  75                  80
   Gln Val His Arg Trp Thr Phe Cys Phe Gln Met Ser Trp Gln Thr Leu
                   85                  90                  95
   Cys His Leu Gly Leu His Tyr Thr Glu Tyr Tyr Leu His Glu Pro Pro
               100                 105                 110
   Ser Val Arg Phe Cys Ile Thr Leu Ser Ser Leu Met Leu Leu Thr Gln
           115                 120                 125
   Arg Val Thr Ser Leu Ser Leu Asp Ile Cys Glu Gly Lys Val Lys Ala
       130                 135                 140
   Ala Ser Gly Gly Phe Arg Ser Arg Ser Ser Leu Ser Glu His Val Cys
   145                 150                 155                 160
   Lys Ala Leu Pro Tyr Phe Ser Tyr Leu Leu Phe Phe Pro Ala Leu Leu
                   165                 170                 175
   Gly Gly Ser Leu Cys Ser Phe Gln Arg Phe Gln Ala Arg Val Gln Gly
               180                 185                 190
   Ser Ser Ala Leu His Pro Arg His Ser Phe Trp Ala Leu Ser Trp Arg
           195                 200                 205
   Gly Leu Gln Ile Leu Gly Leu Glu Cys Leu Asn Val Ala Val Ser Arg
       210                 215                 220
   Val Val Asp Ala Gly Ala Gly Leu Thr Asp Cys Gln Gln Phe Glu Cys
   225                 230                 235                 240
   Ile Tyr Val Val Trp Thr Thr Ala Gly Leu Phe Lys Leu Thr Tyr Tyr
                   245                 250                 255
   Ser His Trp Ile Leu Asp Asp Ser Leu Leu His Ala Ala Gly Phe Gly
               260                 265                 270
   Pro Glu Leu Gly Gln Ser Pro Gly Glu Glu Gly Tyr Val Pro Asp Ala
           275                 280                 285
   Asp Ile Trp Thr Leu Glu Arg Thr His Arg Ile Ser Val Phe Ser Arg
       290                 295                 300
   Lys Trp Asn Gln Ser Thr Ala Arg Trp Leu Arg Arg Leu Val Phe Gln
   305                 310                 315                 320
   His Ser Arg Ala Trp Pro Leu Leu Gln Thr Phe Ala Phe Ser Ala Trp
                   325                 330                 335
   Trp His Gly Leu His Pro Gly Gln Val Phe Gly Phe Val Cys Trp Ala
               340                 345                 350
   Val Met Val Glu Ala Asp Tyr Leu Ile His Ser Phe Ala Asn Glu Phe
           355                 360                 365
   Ile Arg Ser Trp Pro Met Arg Leu Phe Tyr Arg Thr Leu Thr Trp Ala
       370                 375                 380
   His Thr Gln Leu Ile Ile Ala Tyr Ile Met Leu Ala Val Glu Val Arg
   385                 390                 395                 400
   Ser Leu Ser Ser Leu Trp Leu Leu Cys Asn Ser Tyr Asn Ser Val Phe
                   405                 410                 415
   Pro Met Val Tyr Cys Ile Leu Leu Leu Leu Leu Ala Lys Arg Lys His
               420                 425                 430
   Lys Cys Asn
           435
<210> 6
   <211> 289
   <212> PRT
   <213> Artificial Sequence
<220>
   <223> GHS-R1b
<400> 6
   Met Trp Asn Ala Thr Pro Ser Glu Glu Pro Gly Phe Asn Leu Thr Leu
   1               5                   10                  15
   Ala Asp Leu Asp Trp Asp Ala Ser Pro Gly Asn Asp Ser Leu Gly Asp
               20                  25                  30
   Glu Leu Leu Gln Leu Phe Pro Ala Pro Leu Leu Ala Gly Val Thr Ala
           35                  40                  45
   Thr Cys Val Ala Leu Phe Val Val Gly Ile Ala Gly Asn Leu Leu Thr
       50                  55                  60
   Met Leu Val Val Ser Arg Phe Arg Glu Leu Arg Thr Thr Thr Asn Leu
   65                  70                  75                  80
   Tyr Leu Ser Ser Met Ala Phe Ser Asp Leu Leu Ile Phe Leu Cys Met
                   85                  90                  95
   Pro Leu Asp Leu Val Arg Leu Trp Gln Tyr Arg Pro Trp Asn Phe Gly
               100                 105                 110
   Asp Leu Leu Cys Lys Leu Phe Gln Phe Val Ser Glu Ser Cys Thr Tyr
           115                 120                 125
   Ala Thr Val Leu Thr Ile Thr Ala Leu Ser Val Glu Arg Tyr Phe Ala
       130                 135                 140
   Ile Cys Phe Pro Leu Arg Ala Lys Val Val Val Thr Lys Gly Arg Val
   145                 150                 155                 160
   Lys Leu Val Ile Phe Val Ile Trp Ala Val Ala Phe Cys Ser Ala Gly
                   165                 170                 175
   Pro Ile Phe Val Leu Val Gly Val Glu His Glu Asn Gly Thr Asp Pro
               180                 185                 190
   Trp Asp Thr Asn Glu Cys Arg Pro Thr Glu Phe Ala Val Arg Ser Gly
           195                 200                 205
   Leu Leu Thr Val Met Val Trp Val Ser Ser Ile Phe Phe Phe Leu Pro
       210                 215                 220
   Val Phe Cys Leu Thr Val Leu Tyr Ser Leu Ile Gly Arg Lys Leu Trp
   225                 230                 235                 240
   Arg Arg Arg Arg Gly Asp Ala Val Val Gly Ala Ser Leu Arg Asp Gln
                   245                 250                 255
   Asn His Lys Gln Thr Val Lys Met Leu Gly Gly Ser Gln Arg Ala Leu
               260                 265                 270
   Arg Leu Ser Leu Ala Gly Pro Ile Leu Ser Leu Cys Leu Leu Pro Ser
           275                 280                 285
   Leu
<210> 7
   <211> 20
   <212> DNA
   <213> Artificial Sequence
<220>
   <223> Cebador In1-ghrelina F
<400> 7
   tctgggcttc agtcttctcc 20
<210> 8
   <211> 20
   <212> DNA
   <213> Artificial Sequence
<220>
   <223> Cebador In1-ghrelina R
<400> 8
   gttcatcctc tgccccttct 20
<210> 9
   <211> 21
   <212> DNA
   <213> Artificial Sequence
<220>
   <223> Cebador GOAT F
<400> 9
   ttgctctttt tccctgctct c 21
<210> 10
   <211> 21
   <212> DNA
   <213> Artificial Sequence
<220>
   <223> Cebador GOAT R
<400> 10
   actgccacgt ttaggcattc t 21
<210> 11
   <211> 20
   <212> DNA
   <213> Artificial Sequence
<220>
   <223> Cebador GHS-R1b F
<400> 11
   ggaccagaac cacaagcaaa 20
<210> 12
   <211> 21
   <212> DNA
   <213> Artificial Sequence
<220>
   <223> Cebador GHS-R1b R
<400> 12
   agagagaagg gagaaggcac a 21
<210> 13
   <211> 21
   <212> RNA
   <213> Artificial Sequence
<220>
   <223> siRNA (1)
<400> 13
   cacuguuucu ggaaggacat t 21
<210> 14
   <211> 19
   <212> RNA
   <213> Artificial Sequence
<220>
   <223> siRNA (2)
<400> 14
   gagtcctaaa cagactgtt 19